# EUROPEAN PATENT SPECIFICATION

(11) **EP 3 664 936 B1**
(45) Date of publication and mention of the grant of the patent: **27.10.2021**
(21) Application number: 18769182.9
(22) Date of filing: 13.09.2018
(51) Int. Cl.: B02C 18/00, B02C 19/22, B02C 21/02, B02C 23/02, C12P 5/02, C10L 5/44

(54) **METHOD, SYSTEM AND USE THEREOF FOR PROCESSING AN AGRICULTURAL WASTE BIOMASS PRODUCT INTO A GRINDED PRODUCT**
VERFAHREN, SYSTEM UND SEINE VERWENDUNG ZUR VERARBEITUNG EINES LANDWIRTSCHAFTLICHEN BIOMASSEABFALLPRODUKTS ZU EINEM MAHLPRODUKT
PROCÉDÉ, SYSTÈME ET UTILISATION DE CELUI-CI POUR TRAITER UN PRODUIT DE BIOMASSE DE DÉCHETS AGRICOLES EN UN PRODUIT BROYÉ

(30) Priority: 14.09.2017 EP 17191185
(43) Date of publication of application: 17.06.2020
(73) Proprietor: Advanced Substrate Technologies A/S, 8960 Randers SØ (DK)
(72) Inventor: HOFF, Svend, 8300 Odder (DK); OLESEN, Geert, Haugaard, 6100 Haderslev (DK)
(74) Representative: Høiberg P/S
(86) International application number: PCT/EP2018/074776
(87) International publication number: WO 2019/053141

(56) References cited:
- EP-A1- 1 884 563
- CN-A- 106 423 513
- US-A- 6 045 070
- US-A1- 2014 014 748
- US-A1- 2015 020 443
- US-A1- 2015 217 301

## Description

### Field of invention

The present invention relates to a system for processing an agricultural waste biomass product into a grinded product.

### Background of invention

Recently biomass has become increasingly in demand. Biomass may play a key role when replacing fossil energy sources with renewable energy sources. The biomass may be converted into solid fuel such as pellets, liquid fuels, and biogas that are direct substitutes of the fossil based fuels. Further, the biomass may be converted into products that can be used for human or animal nutrition or as fertilizers, or growing medium, for plants, such as substrates for mushrooms, and protein extracts from grass.

Systems for processing an agricultural waste biomass product into a grinded product are known in the art, for example from documents US 2014/014748 A1, EP 1 884 563 A1, US 6 045 070 A, CN 106 423 513 A and US 2015/020443.

A major disadvantage with biomass is the initial transport logistics from the production site, e.g. the field, farm or the stable, to the storage or the plant, and the associated susceptibility to weather conditions. For example litter from animals or harvested cut straw may be spread across the field to dry before it is suitable for collection, storage, and/or transport.

Further, harvested biomass crop has irregular shapes, sizes, compaction, and a high volume, and a low energy density. Thus, after harvesting it is necessary to gather or collect, the harvested biomass, and optionally to further cut and/or compact the material into harvest units with sizes and compaction that is suitable for storage, transportation and/or further processing. By the term "harvest unit" is meant harvested crops that have been collected into a more concentrated bulk, in contrast to the original harvested crops. For example harvested straw may be subjecting to baling, where the baling compresses, or densifies, the straw, and the bales constitute harvest units that are easier, or easily transported and stored, thereby improving the logistics.

When biomass is used as feed stock in a biogas plant, it is further essential that the biomass has a size and shape that facilitate the chemical conversion of the biomass to biofuel. For example, it may be essential that the biomass feed stock have a small and uniform size, and is compacted in such a degree that the surface area of the feed stock is sufficiently exposed for the chemical reactions to occur.

Thus, after harvesting, collection, and/or transport of the harvest units or biomass waste, the biomass is typically cut to sizes suitable for chemical treatment. The cutting is conventionally carried out in a chopper followed by a hammermill.

Harvested biomass and other types of agricultural waste products are particularly susceptible to the weather conditions, since the first collection and storage steps are outdoor. As is known from conventional agriculture, wet biomass is susceptible to rot and degradation of the biomass sugars. In addition, wet biomass is problematic to process in a hammermill, and use of wet biomass in biofuel conversion systems are known to increase the risk of clogged pipes, and floating layers i.e. inefficient material transport in the system. Thus, harvested wet biomass and other types of wet agricultural waste products are typically lost, or alternatively, an additional drying step is essential before storage and/or use, thus further complicating the logistics.

There is therefore a need for systems that can decrease the loss associated with wet biomass, such as wet harvested biomass and other types of agricultural waste products. Further, there is a need for systems that can improve the biomass logistics, and make biomass harvesting less susceptible to weather conditions.

### Summary of invention

The invention relates to a system in accordance with claim 1, to a method in accordance with claim 14 and to the use of the system of any of claims 1 to 13 in accordance with claim 14.

Dependent claims refer to preferred embodiments of the invention.

The present disclosure provides a system that may decrease the biomass loss associated with wet biomass. Thus, the present disclosure provides a more efficient biomass production. Further, the system may improve the biomass logistics, and make biomass harvesting less susceptible to weather conditions. The provided system may further improve logistics by being used as a stationary or mobile or moveable system. By the term "mobile" or "moveable" is meant that the system may be self-propelled and/or trailed by e.g. a tractor.

The present disclosure provides a system for processing any types of agricultural waste products into a grinded product, where the grinded product is suitable as a substrate, or feed stock, for biogas, and/or as a primary growth media for production of plants, such as vegetables production and/or mushroom production.

Examples of agricultural waste products include harvested biomass, such as straw, grass, low quality grass silage, compromised hay, deep litter from e.g. poultry, cattle, and horses, leaves and smaller branches, feather from poultry, and any combinations thereof. Preferably, the biomass is organic or eco-friendly biomass, such as rice straw, elephant grass, e.g. pennisetum purpureum, saccharum ravennae, miscanthus fuscus, and biomass from willow trees. Eco-friendly biomasses are plants providing an environmental improvement to the surroundings, e.g. by attracting, collecting and/or concentrating nutrients into the plant surroundings.

A first aspect of the invention relates to a system **100** for processing an agricultural waste biomass product into a grinded product, according to claim 1. Said system comprising:
- a cutting and mixing unit **6** configured for cutting and mixing harvested biomass to a first uniform size and blend,
- a grinder unit **9** configured for cutting biomass to a second uniform size,
wherein the cutting and mixing unit is in fluid communication with the grinder unit through a first transport unit **7** configured for transporting the biomass from the cutting and mixing unit to the grinder unit.

A second aspect of the invention relates to a method for processing an agricultural waste biomass product into a grinded product, according to claim 14. Said method comprising the steps of:
- providing a harvested agricultural waste biomass,
- cutting and mixing the biomass into a first uniform size and blend within a cutting and mixing unit **6** configured for cutting and mixing harvested biomass to a first uniform size and blend,
- grinding the cut and mixed biomass into a second uniform size within a grinder unit **9** configured for cutting biomass to a second uniform size,
whereby a grinded product suitable as a substrate for a biogas plant and/or as primary growth substrate for a plant, such as mushroom, and/or as substrate for producing green protein extract.

The agricultural waste biomass can be selected from the group of: straw, grass, low quality grass silage, compromised hay, deep litter from poultry, cattle, and/or horses, leaves and smaller branches, feather from poultry, and any combinations thereof. In a further preferred embodiment, the biomass is selected from the group of organic or eco-friendly biomass, such as rice straw, elephant grass, e.g. pennisetum purpureum, saccharum ravennae, miscanthus fuscus, and biomass from willow trees.

In another preferred embodiment, the second uniform size is smaller than the first uniform size. By the term "uniform size" is meant a size distribution with a narrow size distribution, such as a distribution where 95% or 85% of the sizes are within 5, 10, 20, 30, 40, or 50% of the average size. In a further embodiment, the second uniform size corresponds to a diameter between 1 to 40 mm, more preferably between 6 to 24 mm, and most preferably between 12 to 16 mm.

Further, according to the present disclosure, the method according to the second aspect of the invention is carried out within the system according to the first aspect of the invention. For example, the steps comprising the cutting and mixing unit and the grinder unit are carried out within the system according to the first aspect of the invention. Thus, the cutting and mixing unit and the grinder unit of the method, are comprised within the system according to the first aspect of the invention.

A third aspect of the invention, as defined in claim 15, relates to the use of the system according to the first aspect for processing an agricultural waste biomass product into a grinded product, suitable for a substrate for a biogas plant and/or as primary growth substrate for a plant, such as mushroom, and/or as substrate for producing green protein extract, or as feedstock unit for one or more a biogas plant(s).

### Description of Drawings

The invention will in the following be described in greater detail with reference to the accompanying drawings.
Figure 1 shows an embodiment of a system **100** for processing agricultural waste product biomass directly into a grinded product, as seen from the top.
Figure 2 shows an embodiment of a system **100** for processing agricultural waste product biomass directly into a grinded product, as seen in profile.
Figure 3 shows an embodiment of a grinder unit according to the present invention, and a grinded product exemplified as wood chips.
Figure 4 shows an embodiment of a cutting and mixing unit, comprising two oppositely rotating screws.
Figure 5 shows an embodiment of cutting and mixing unit of the type chopper and mixer from the Italian company SEKO, which is a Chopping-Mixing-Wagon from the Samurai 5 series.
Figure 6 shows an embodiment of a trailed cutting and mixing unit.
Figure 7 shows an embodiment of a transport unit configured for separating a liquid product from a solid product, such as a grinded biomass product. The embodiment shows a single screw press for separation of fresh cuttet grass, grinded grass, or other types of biomass into a liquid fraction, or juice fraction, and a fiber fraction, or press cake fraction. (A) shows the part of the screw press where biomass is fed in from the grinder unit and (B) shows the part of the screw press where the fiber or press cake exit the screw press unit with a dry matter content of up to 35%.

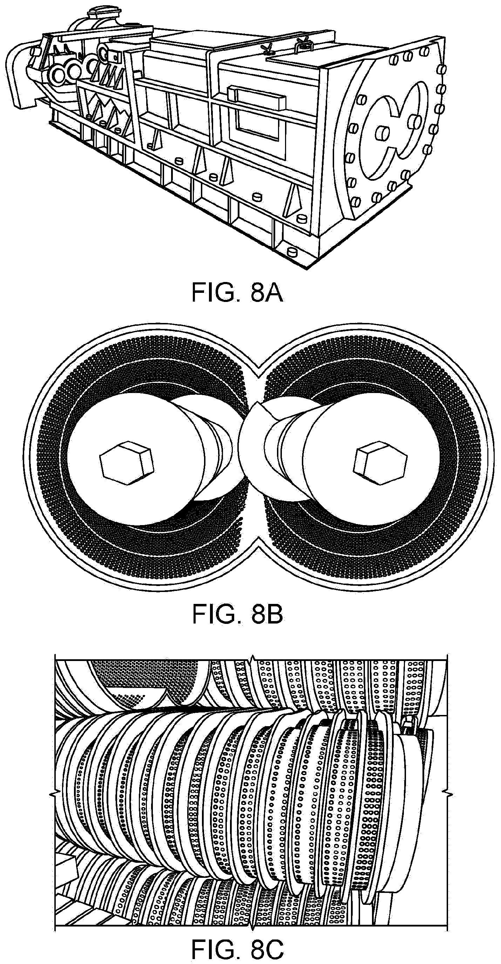

Figure 8 shows an embodiment of a transport unit configured for separating a liquid product from a solid product, such as a grinded biomass product. The embodiment shows a twin screw press for separation of fresh cuttet grass, grinded grass, or other types of biomass into a liquid fraction or juice and a fiber fraction or press cake, where the fiber or press cake exit the twin screw press with a dry matter content of up to 50 wt%. (A) shows a twin screw press unit seen from outside, (B) shows the two screws having opposite rotation directions, where one is turning left and the other is turning right, and (C) shows the filter with holes for elimation of the juice.
Figure 9 shows an embodiment of a biogas plant, where the grinded product from e.g. a mobile or stationary system according to the invention is transferred to the nitrogen steamer unit (indicated as "N-steamer").
Figure 10 shows an embodiment of a moveable system **100** for processing harvested biomass into a grinded product. In the embodiment, the harvested biomass is temporary stored **1** on e.g. a field or farm, and the operation of the mobile system, including the grinding, takes place on the location, where the harvested biomass is temporary stored **1** on e.g. the farm.
Figure 11 shows a further detailed embodiment of a moveable system **100** from a top view.
Figure 12 shows an embodiment of the moveable system **100** from a side view.
Figure 13 shows an embodiment of a stationary system **100** according to the present invention.
Figure 14 shows an embodiment of the moveable system **100** according to the present invention, in fluid communication with one or more biogas plants **17.**
Figure 15 shows an embodiment of the invention, where the system **100** is a stationary unit, optionally directly connected to a biogas plant **17.**
Figure 16 shows an embodiment of the moveable system according to the present invention, adapted for processing fresh to grass juice for green protein production.

### Detailed description of the invention

The invention is described below with the help of the accompanying figures. It would be appreciated by the people skilled in the art that same feature of component of the device are referred with the same reference numeral in different figures. A list of the reference numbers can be found at the end of the detailed description section.

By the term "agricultural waste products" is meant harvested biomass, such as straw, grass, low quality grass silage, compromised hay, deep litter from e.g. poultry, cattle, and horses, leaves and smaller branches, feather from poultry, and any combinations thereof. Further examples of agricultural waste products are plants or biomass selected from the group of organic or eco-friendly biomass, such as rice straw, elephant grass, e.g. pennisetum purpureum, saccharum ravennae, miscanthus fuscus, and biomass from willow trees.

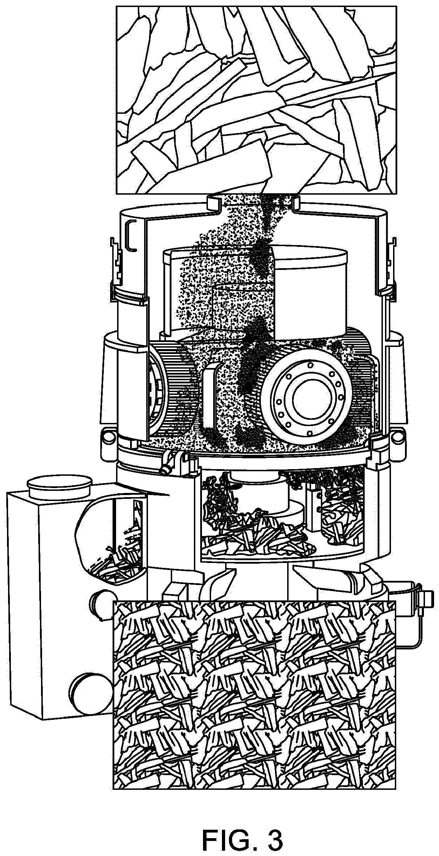

Agricultural waste products, such as harvested straw or litter from animals, are typically collected and coarsely processed, i.e. e.g. coarsely cut, mixed, and or compacted, at the production site, i.e. the field, stable or farm, to make it suitable for particularly transport. After transport to a storage site, the collected biomass is further processed to make it suitable for storage, conversion into biofuel, and/or conversion into biomass products, such as growth media. An example of a further processed biomass that is suitable for storage, conversion into biofuel, and/or conversion into biomass products is a grinded product. By the term "grinded product" is meant biomass constituting of small and uniform pieces, such as wood chips as illustrated in Figure 3. The further processing may include steps such as further drying, cutting, mixing, and/or compaction. For example, grinded biomass is suitable for conversion into biofuel.

The present invention provides a more efficient and compact system for processing the agricultural waste biomass product from the production site and directly into the products suitable for storage, biofuel, and/or biomass products. Thus, the present invention provides a more simple system for processing agricultural waste products, such as a system where the transport logistics are simplified.

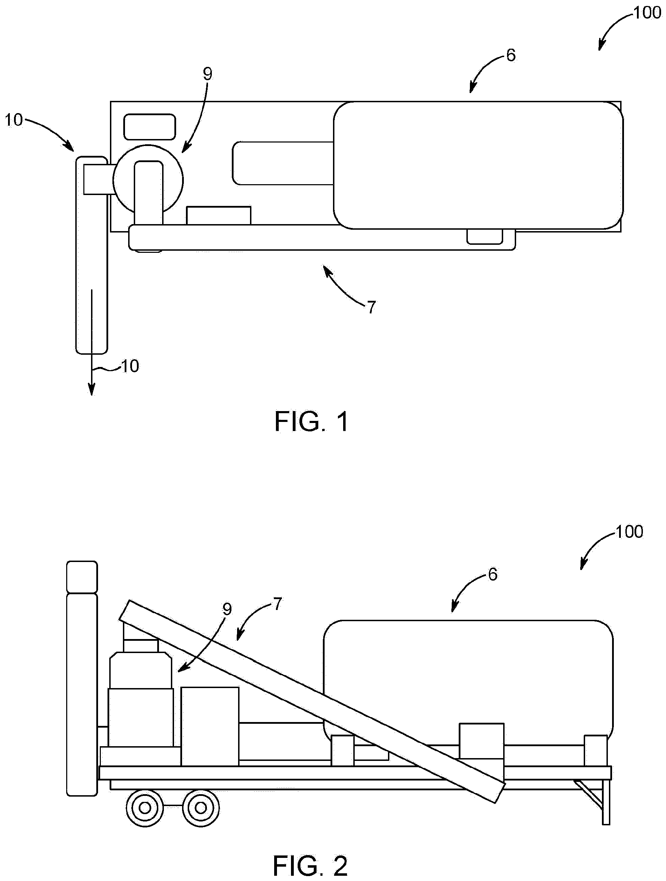

Figures 1 and 2 show an embodiment of a system **100** for processing agricultural waste product biomass directly into a grinded product. The agricultural waste product is first subjected to a cutting and mixing unit **6,** where the waste product is mixed and cut to a first uniform size and blend. The cut and mixed waste product is then transferred via a first transport unit **7** to a grinder unit **9,** where it is converted into a grinded product, having a second uniform size that is finer in size than the first uniform size, and which optionally is further mixed and packed.

### Cutting and mixing unit

The cutting and mixing unit is configured for cutting and mixing agricultural waste products in the raw initial form, such as straw, bales, grass, low quality grass silage, compromised hay, and litter from animals. For example, the cutting and mixing unit may be configured for cutting and mixing agricultural waste products of organic or eco-friendly biomass in the raw initial form, such as rice straw, elephant grass, e.g. pennisetum purpureum, saccharum ravennae, miscanthus fuscus, and parts from willow trees.

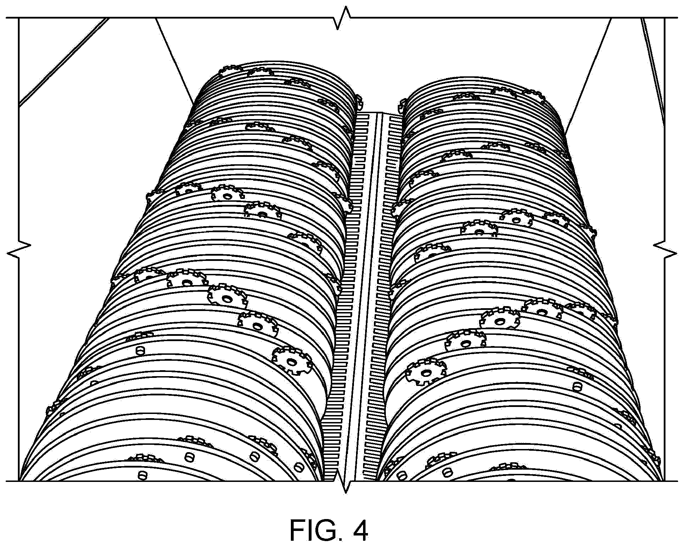

An example of a cutting and mixing unit and the operational principle is shown in Figure 4. Figure 4 shows how the cutting and mixing mechanism is based on two rotating snecks, or screws, with knifes. The snecks are rotating slowly, and in opposite directions, e.g. one sneck is turning left and the other right. This facilitates that the waste product, or biomass, entering the cutting and mixing unit is mixed and at the same time the knifes will cut it in smaller pieces.

Advantageously, the cutting and mixing unit is working as a semi-batch process, such that the time for mixing and cutting may be sufficiently controlled before the biomass is further transferred to the grinder.

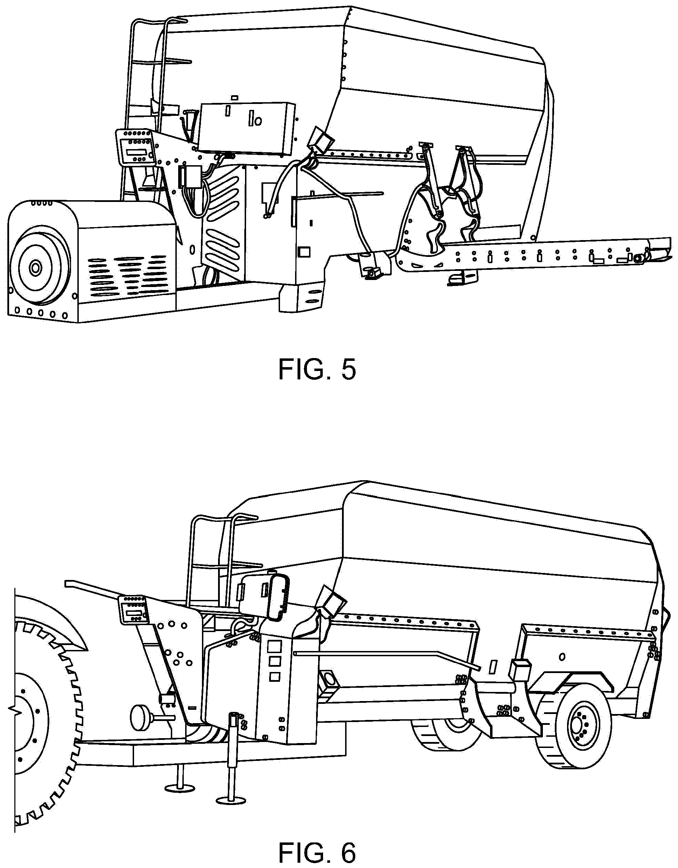

An example of a cutting and mixing unit, or a collection unit is a chopper, or a chopper and mixer, as illustrated in Figure 5. The exemplified chopper and mixer is from the Italian company SEKO, and is a Chopping-Mixing-Wagon from the Samurai 5 series.

The type of cutting and mixing unit affects the degree and quality of the cutting and mixing of the harvested biomass, and thus, the resulting first uniform size and blend of the biomass after the cutting and mixing unit. A first uniform size and blend that is advantageous as feedstock for a grinder unit, may be obtained with a cutting and mixing based on rotating screws, such as the chopper and mixer.

In an embodiment of the invention, the cutting and mixing unit comprises two rotating screws, wherein the screws are configured to rotate in opposite directions, and preferably the cutting and mixing unit is selected from the group of chopper and chopper and mixer.

The chopper and mixer is conventionally used for forages, such as straw and coarse fodder for dairy cows, fattening cattle, and sheep-and-goat livestock, as well as other types of fibrous products. The coarse fodder may for example include: beets, ensilage, grass, grass silages, corn, maize, and baled or loose straw.

Advantageously, a chopper and mixer has a volume capacity, such as from 5 to 30 m³, whereby it may be adapted for being moveable, e.g. self-propelled or operated trailed, or stationary operation. Figure 5 shows an embodiment of a stationary collection unit, and Figure 6 a trailed version, where the collection unit is attached to a truck body. A moveable, or mobile, and self-propelled version can further be obtained by including an electric motor.

In an embodiment of the invention, the cutting and mixing unit has a volume capacity between 1 to 50 m³, more preferably between 3 to 40 m³, or 10 to 20 m³, and most preferably is from 5 to 30 or 5 to 33 m³.

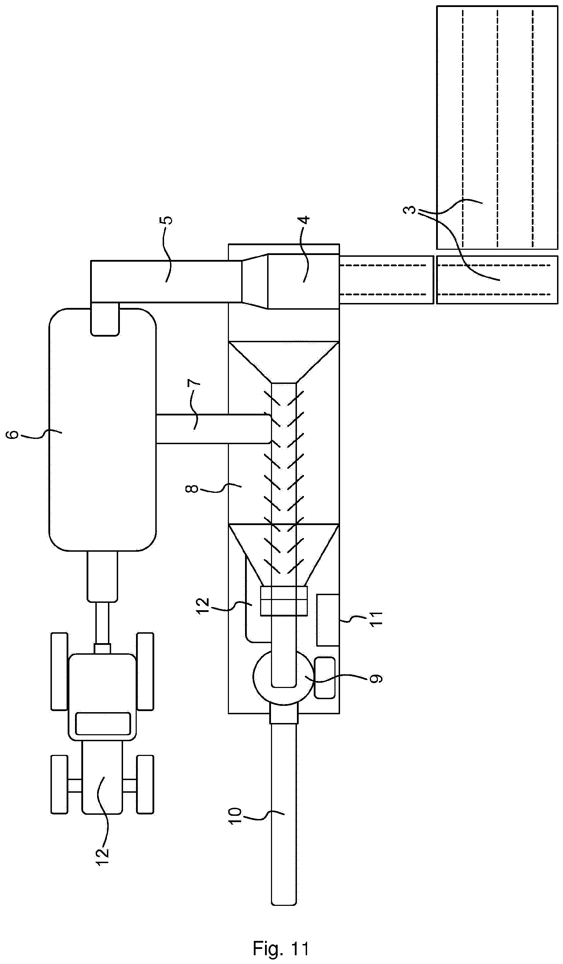

The cutting and mixing unit may operate more efficiently, when the load of biomass within the unit is controlled. Thus, advantageously, a chopper and mixer includes feedstock control means, such as a silage tiller, adapted to control the feed into the cutting and mixing unit, as illustrated in Figures 11-12 as element **5.**

In an embodiment of the invention, the system further comprises a second transport unit **5** configured for transporting the harvested biomass to the cutting and mixing unit. In an embodiment of the invention, the cutting and mixing unit is in fluid communication with a second transport unit **5** adapted as a feedstock control means, such as a feeder unit or a silage tiller. Optionally the second transport unit is a band conveyor with an inclination between 0-45 degrees, more preferably between 10-30 degrees, and most preferably between 15-20 degrees.

### First transport unit

The cut and mixed product is transferred from the cutting and mixing unit to the grinder unit, via the first transport unit. To obtain an efficient and clean transfer with minimum wasted material, it is advantageous that the first transport unit is of a conveyor type, such as a conveyor selected from the group of: screw-, chain-, flight-, and pneumatic conveyors, and more preferably is a screw conveyor.

In an embodiment of the invention, the first transport unit is a conveyor, preferably selected from the group of: screw-, chain-, flight-, and pneumatic conveyors, and more preferably is a screw conveyor.

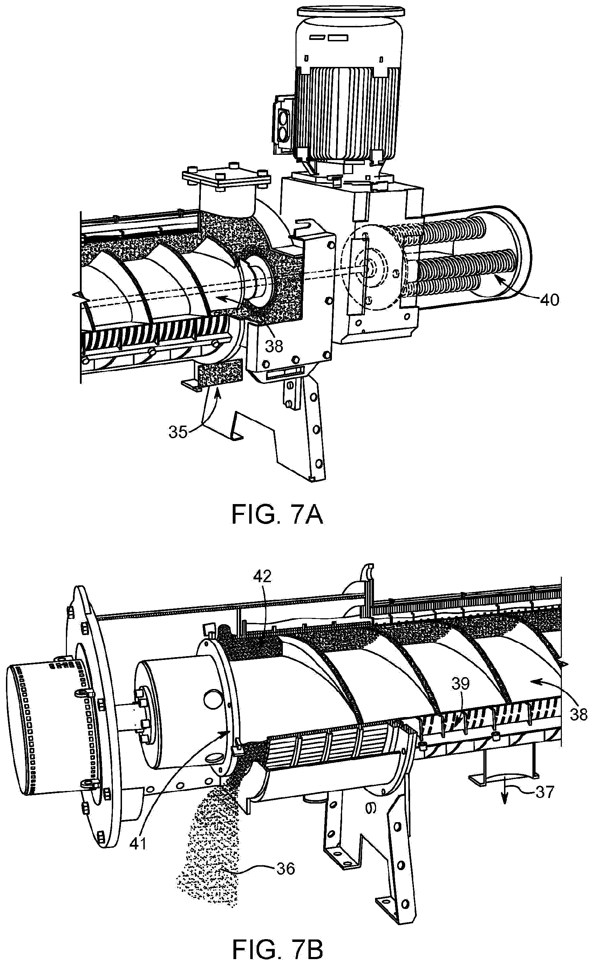

Optionally, the first transport unit is a screw conveyor, and optionally the screw conveyor is further configured for separating solid and liquid biomass during the transport of the biomass as exemplified in Figures 7 and 8. The screw conveyors illustrated in Figures 7-8 are further described in the section "moveable system for grass".

Figure 7A shows the part of the screw conveyor where biomass is fed from the cutting and mixing unit and into the first transport unit, i.e. biomass inlet **35.** Figure 7B shows the part of the conveyor where the transported biomass may exit the transport unit, either as solid matter from biomass solid outlet **36,** and/or as liquid from biomass liquid outlet **37.**

In some embodiments, it is advantageous that the screw conveyor is configured to separate solid and liquid biomass during the transport of the biomass, thereby improving the efficiency of the system, and the utilization degree of the original raw harvested biomass. The solid biomass exiting the first transport unit, may be further treated in a grinder unit, whereas the liquid biomass may be used in a biogas plant.

In an embodiment of the invention, the first transport unit is configured to separate solid and liquid biomass during the transport of the biomass.

Figure 7B shows how the separation may be efficiently carried out by a mechanical process. The screw snail **38** is seen to rotate within an outer tube, wherein a grid or mesh **39** is placed in a concentric distance from the tube wall. Thus, as the biomass feedstock is transported by the screw snail, the liquid part of the biomass will be able to exit through the mesh and subsequently the liquid outlet **37,** while the solid part of the biomass is transferred to the end channel **42,** defined by a disc **41** at the end. The solid biomass may exit the transport unit through the solid outlet **36,** where the outlet may be generated by displacing the disc **41** in the axial direction of the rotation axis. The axial displacement may be obtained by pressure means **40** placed at the opposite end of the transport unit as illustrated in Figure 7A. The pressure means may be manually or pneumatically operated.

### Grinder unit

The grinder unit is configured to produce a grinded product, i.e. small and uniform pieces that are suitable for storage, conversion into biofuel, and/or conversion into biomass products. Examples of a grinded biomass product include crushed wood chips, wood shavings, sawdust and other lumpy biomass, as illustrated in Figure 3. Thus, the grinded product will have a second uniform size that is smaller than the first uniform size of the biomass obtained after the cutting and mixing step. The second uniform size further implies that the grinded product facilitates a different packing degree of the grinded pieces, compared to the packing degree of the biomass after cutting and mixing.

A grinded biomass product may further be optimal for conversion into biofuel and biomass products, such as a plant growth media. Due to the uniform and small size of the grinded product, the pieces will be volumetrically packed in a certain degree and with an exposed surface area making it ideal for chemical treatment and conversion. When biomass is used as feed stock in a biogas plant, it is essential that the biomass has a size and shape that facilitate the chemical conversion of the biomass to biofuel. For example, it may be essential that the biomass feed stock have a small and uniform size, and is compacted in such a degree that the surface area of the feed stock is sufficiently exposed for the chemical reactions to occur. For example, converting biomass to biogas and substrates for plants involves the chemical processing step of treating the biomass with a steam comprising nitrogen to separate the fermentable sugars from the lignin within the biomass. The efficiency of the nitrogen steaming process will thus depend on the contact time and degree of contact surface between the steam and the biomass. The contact surface will depend on the size and shape (and thus inherently the surface area of the biomass) as well as the compaction degree (i.e. the access of the steam to the surface of the biomass).

The size, shape, and compaction degree of the resulting grinded biomass material will depend on the type of grinder apparatus. Conventionally, a hammermill is used to produce a grinded biomass suitable for nitrogen steaming, such as to convert straw into a biogas substrate.

In a hammermill, dry biomass is fed to the mill chamber, where it is struck by ganged hammers attached to a shaft that rotates at high speed inside the grinding chamber. The biomass material is then crushed and shattered to a reduced size by the hammer impacts and collisions with the grinding chamber walls. The discharge opening of the grinding chamber may be a screen with a defined mesh size, such that material with sizes below will be allowed to pass and be discharged, and coarser material will be further grinded.

However, the operational principle of a hammermill implies that for the hammermill to operate efficiently, it is essential that the biomass is dry. Moist or wet biomass will cause the grinding process to be inefficient and/or clogging of the hammermill.

The inventors surprisingly found that a grinder unit according to the present invention may replace a hammermill, when processing biomass to a size appropriate for chemical processing, particularly nitrogen steaming. Particularly it was seen that the system comprising the grinder unit according to the present invention may result in a grinded biomass with size, shape, and compaction degree resulting in a further improved efficiency of the nitrogen steaming process. For example, the grinder unit according to the present invention in combination with N-steaming of straw were seen to result in an increased biogas yield of 45-60 m3 CH₄ per 1000 kg, corresponding to 55-70 m3 CH₄ per 1000 kg dry matter.

It was further seen that the grinder unit according to the present invention resulting in a more efficient grinding process, and in particular, comparable or more efficient grinding was obtained on wet or moist biomass. Thus, grinded biomass with size, shape, and compaction degree suitable for a chemical vapour process, such as nitrogen steaming, may be produced from both wet or moist biomass.

An embodiment of a grinder unit according to the present invention is shown in Figure 3. The cut and mixed biomass is entered into the top of the grinder and then crashed and pressed through the matrice by three or more rotating koller heads/wheels. Thus, the size and shape of the grinded product exiting the grinding unit will depend on the wheels as well as the matrice properties, and especially the shape and size of the holes, or openings, in the matrice.

In an embodiment of the invention, the grinder unit is not a hammermill. In another embodiment, the grinder unit is configured to operate on moist or wet biomass.

In a further embodiment, the grinder unit comprises two or more rotating wheels configured for cutting biomass, preferably comprising three rotating wheels, and a matrix comprising multiple openings configured for transferring grinded biomass with a size below the size of the openings. Thus, grinded biomass may be transferred through the openings, and thereby discharged or emitted out of the grinder unit. In a further embodiment, the openings have a diameter between 1 to 40 mm, more preferably between 6 to 24 mm, and most preferably between 12 to 16 mm.

### Systems

The grinded product may be transferred away from the grinder unit via a third transport unit **10** as indicated in Figure 1. Similar to the first and/or second transport unit, the third transport unit may advantageously be of a conveyor type. The third transport unit facilitates that the grinded product may be unloaded at any location.

In an embodiment of the invention, the system further comprises a third transport unit **10,** such as a conveyor, configured for transferring the grinded biomass from the outlet of the grinder unit.

The system according to the invention comprising the cutting and mixing unit, the first transport unit, and the grinder unit, may be stationary and e.g. connected directly to a nitrogen steaming plant. The system may further be stationary and connected to a storage configured to directly provide biomass to a nitrogen steaming plant, such as a walking floor container in fluid communication with a biogas plant.

Alternatively, the system according to the invention is a mobile, or moveable, and separate system from the nitrogen steaming plant, which may be detachably connected to one or more nitrogen steaming plant(s) or one or more storage(s) connected to the nitrogen steaming plant.

In an embodiment of the invention, the system is configured to be mobile, moveable, or stationary.

Figure 2 shows an embodiment of a mobile, or moveable, system **100,** shown in a cross-sectional view. The cutting and mixing unit **6** is in fluid communication with the grinder unit **9** via a first transport unit **7.**

Optionally, as indicated in Figure 2, the first transport unit is a conveyor with an inclination between 0-90 degrees, more preferably between 10-45 degrees, and most preferably between 25-35 degrees.

The mobile system may be operated by one person, where the person feeds the cutting and mixing unit with biomass. The system **100** unit may further comprise a control panel (SRO), a power unit, such as a motor generator, with a capacity of circa 200 kW and a tractor with 150 HP on diesel or preferably on biomethane or a mix of biomethane and diesel.

The movable system may be operated by one person per shift and with a capacity of 3,5 ton dry matter per hour in operation.

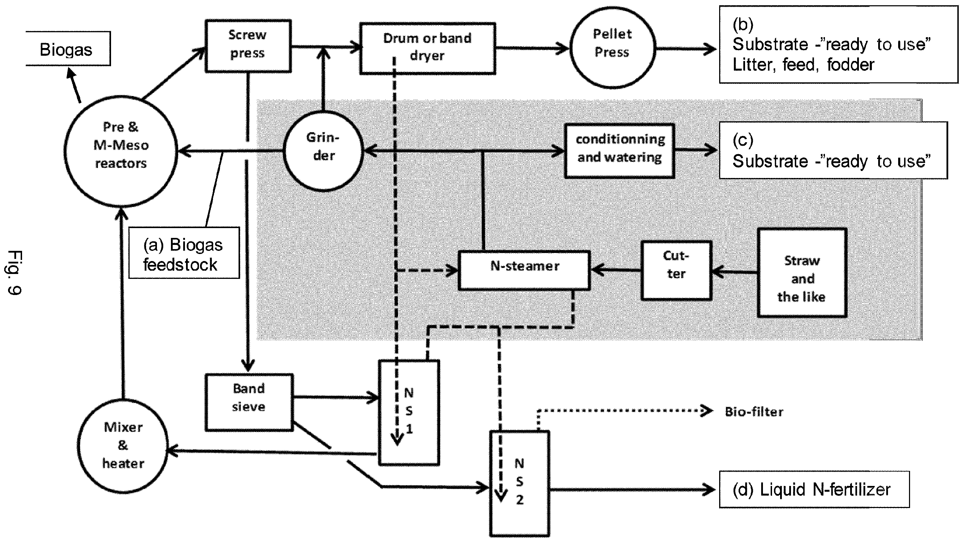

Figure 9 shows an embodiment of a biogas plant. The grinded product from e.g. a mobile or stationary system according to the invention is transferred to the nitrogen steamer unit (indicated as "N-steamer" in Figure 9). The nitrogen steamer may optionally be operated using the warm and moist steam comprising nitrogen from a nitrogen stripper unit, and/or separation- or drying unit. From the nitrogen steamer, the grinded and steamed material may be fed to a biogas reactor.

Optionally, other sources of biomass may be added to the grinded product before being introduced into the N-steamer. Examples of other sources of biomass include: agricultural waste such as grass silage, animal waste such as deep litter from e.g. poultry, cattle, and horses, as well as organic or eco-friendly biomass, such as rice straw, elephant grass, e.g. pennisetum purpureum, saccharum ravennae, miscanthus fuscus, and biomass from willow trees.

A mobile, moveable, or stationary system, advantageously comprises one or more of the following elements, to facilitate the operation: a buffer and feeder unit **8,** a control unit **11,** a power supply unit **12,** a feeder unit **3,** and an eliminator unit **4.**

The buffer and feeder unit is advantageously configured for controlling the supply and transfer of the biomass from the cutter and mixer unit to the grinder unit. For more efficient operation of the grinder, the load of biomass within the grinder is advantageously controlled. Similarly, the cutting and mixing unit is operating more efficiently if the load within the unit is controlled, and the amount of other materials, such as ropes, from e.g. the bales, or stones are minimized. Thus, advantageously, the system comprises one or more eliminator unit(s), configured for separating the biomass from other materials, such as rope, stone, metal, and other heavy particles. Advantagously, the buffer and feeder unit is connected with an eliminator unit, and further advantageously the second transport unit is connected with an eliminator unit. For example, the buffer and feeder unit advantageously is fluidly connected with an eliminator unit, such that stone, rope and other heavy particles are removed before entering the grinder. The eliminator may for example be mounted at the top of the buffer and feeder unit, and be a top-mounted eliminator.

In an embodiment of the invention, the system comprises a buffer and feeder unit **8** configured for supplying the biomass to the grinder unit. In a further embodiment, the system further comprises a control unit **11,** such as a a panel or a SRO. In a further embodiment, the system further comprises a power supply unit **12,** such as a motor-generated plant or a tractor.

In a further embodiment, the system further comprises a feeder unit **3** configured for transferring straw in bales or loose. In a further embodiment, the system further comprises an eliminator unit **4** configured for separating the biomass from other materials, such as rope and stones, metal and other heavy particles. In a further embodiment, the system comprises multiple eliminator units.

### Moveable systems

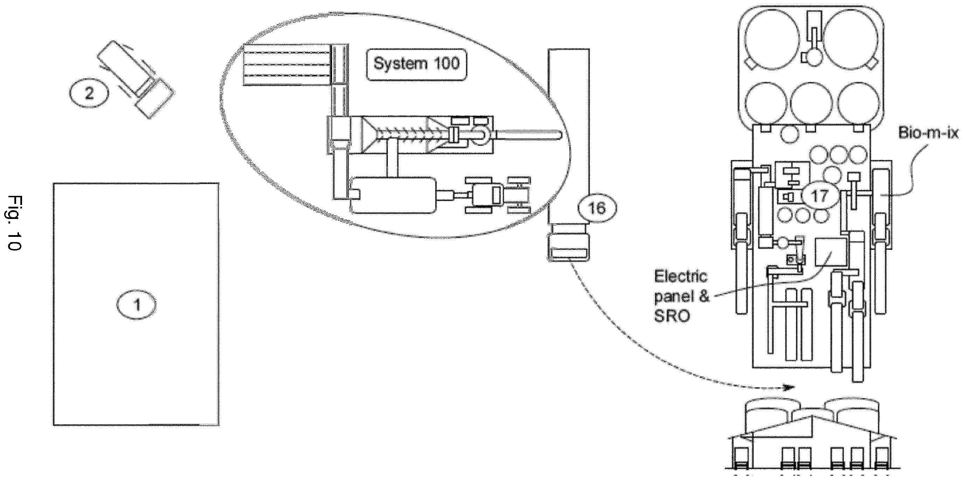

Figure 10 shows an embodiment of a moveable system **100** for processing harvested biomass into a grinded product. In the embodiment, the harvested biomass is temporary stored **1** on e.g. a field or farm, and the operation of the mobile system, including the grinding, takes place on the location, where the harvested biomass is temporary stored **1** on e.g. the farm.

In an embodiment of Figure 10, the harvested biomass may be straw in bales or loose. In a further embodiment, the harvested biomass is selected from the group of: straw, grass, animal biomass, and any combinations thereof. In a further embodiment, the the harvested biomass is either in a dry, wet, or moist form. In a further embodiment, the harvested biomass is organic or eco-friendly biomass, such as rice straw, elephant grass, e.g. pennisetum purpureum, saccharum ravennae, miscanthus fuscus, and biomass from willow trees.

From the temporary storage **1,** the harvested biomass is moved by loader **2** and loaded into the moveable system **100.** In the embodiment, the system comprises the components with reference number **3** (feeder for straw in bales or loose), **4** (eliminator for removing ropes, stones, and other heavy particles from the biomass e.g. straw), **5** (second transport unit for transporting the biomass to the cutting and mixing), **6** (cutting and mixing unit), **7** (first transport unit), **8** (buffer and feeder unit) optionally with a top-mounted eliminator for separating the biomass from other materials, such as rope and stones, metal and other heavy particles, **9** (grinder unit), **10** (third transport unit), **11** (control panel, such as SRO), and **12** (power supply, such as a motor-generator plant, or a tractor).

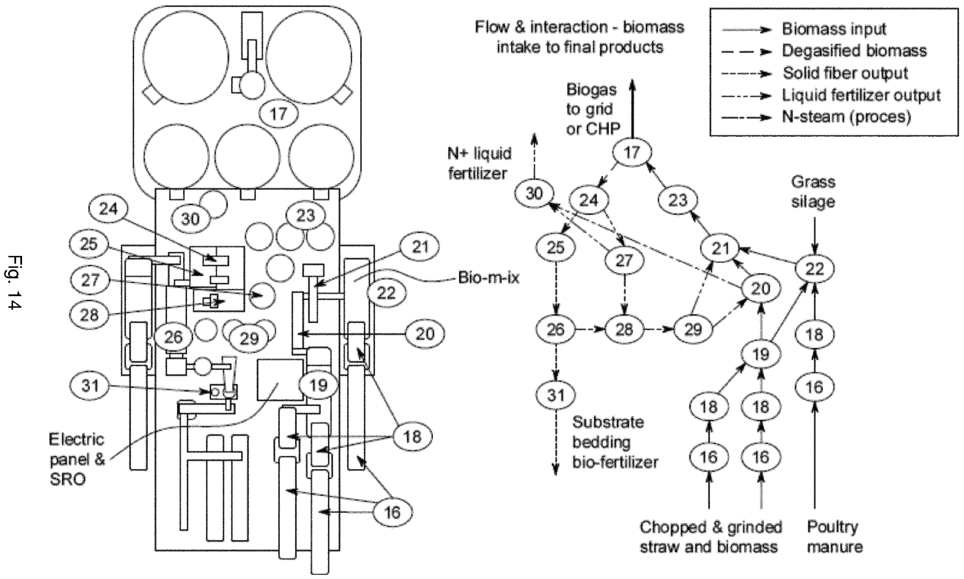

From the system **100,** the grinded product may be transferred to a buffer storage and/or a transport unit **16,** and e.g. transported to a biogas plant **17.** An embodiment of a biogas plant comprises the elements with reference number **18** (unloader/loader unit), **19** (buffer and feeder unit), **21** (power feed), **22** (bio mixer), **23** (heater unit), **24** (separation or screw press), **25** (fiber or solid outlet), and **27** (reject liquid outlet). Preferably, the biogas plant further comprises **20** (N-steamer unit), **26** (dryer unit), **28** (water ring pumps), **29** (N-stripper unit), **30** (N-absorber unit), and **31** (compactor or pellet press unit). A detailed embodiment of a biogas plant is shown in Figure 14.

Thus, in an embodiment the grinded biomass is compacted and stored on location and then loaded in transport units and transported to biogas plants when needed or convenient.

A further detailed embodiment of a moveable system **100** from a top view is shown in Figure 11. The harvested biomass crops are taken from the temporary store **1** by a loader **2** (not shown in Figure 11), and loaded into a feeder unit **3,** and fed to an eliminator unit **4,** which is a device that cut and eliminate ropes, loosen the straw and eliminate stones, metal and heavy particles. The remaining loose straw is transferred or fed by the second transport unit **5** into a cutting and mixing unit **6,** where the crops are cut and mixed to a more uniform size and blend. The cut and mixed product is then transferred via the first transport unit **7,** into a buffer and feeder unit **8,** optionally with a top-mounted eliminator for separating the biomass from other materials, such as rope and stones, metal and other heavy particles, from which it may be transferred further to the grinder unit **9,** and further out of the system via a third transport unit **10.** For the moveable system to operate on location, such as any place on a farm, it is further necessary that the system comprises a control unit **11,** such as a panel or a SRO, and a power supply unit **12,** such as a motor-generated plant or a tractor.

In an embodiment of the invention, the system is configured as a moveable system comprising: a feeder unit **3,** an eliminator unit **4,** a second transport unit **5,** a cutting and mixing unit **6,** a first transport unit **7,** a buffer and feeder unit **8,** a grinder unit **9,** a third transport unit **10,** a control unit **11,** and a power supply unit **12.**

Ropes, stones and heavy particles are typically not present in all types of harvested biomass, such as grass silage, fresh cut grass, and deep litter. Thus, depending on the type of harvested biomass, the feeder unit and eliminator unit may be optional. For example, grass silage, fresh cut grass, and deep litter may advantageously be loaded directly into the cutting and mixing unit **6** via a second transport unit **5.**

In a preferred embodiment of the invention, the system is configured as a moveable system comprising: a second transport unit **5,** a cutting and mixing unit **6,** a first transport unit **7,** a buffer and feeder unit **8,** a grinder unit **9,** a third transport unit **10,** a control unit **11,** and a power supply unit **12.**

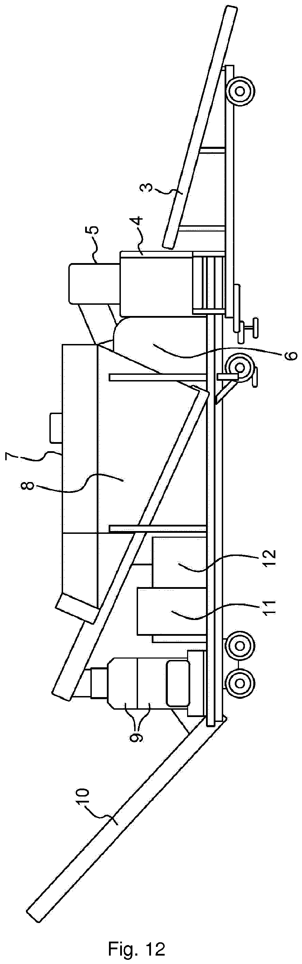

Figure 12 shows an embodiment of the moveable system **100** from a side view, or in profile. In the embodiment, the feeder unit **3** is exemplified as two conveyors in communication, where the harvested biomass or harvest units are placed on the first conveyor by a loader, and the second conveyor feed the bales into the eliminator unit **4.**

In an embodiment of the invention, the eliminator unit **4** is divided into 3 sections, where the first section is configured for cutting and eliminating the ropes e.g. by means of cutters, the second section is configured for loosen the straw e.g. by means of rotators, and the third section is configured for separating stones, metal, and heavy particles from the loosen straw by e.g. magnet means and/or air flow, e.g. by blowing the straw into the second transport unit **5.**

In an embodiment of the invention, the eliminator unit comprises one or more sections, and wherein at least one section comprises magnetic means or means for providing an air flow means.

In an embodiment of the invention, the second transport unit **5** is a band conveyor with an inclination between 0-45 degrees, more preferably between 10-30 degrees, and most preferably between 15-20 degrees.

In an embodiment of the invention, the cutting and mixing unit **6** is a tractor driven fodder mixer wagon, similar to a wagon typically used on bigger cattle farms and exemplified in Figure 6. The chopper and mixer is conventionally used for forages, such as straw and coarse fodder for dairy cows, fattening cattle, and sheep-and-goat livestock, as well as other types of fibrous products. The coarse fodder may for example include: beets, silage, and baled or loose straw. Other types of dry or wet forages may also be processed within the cutting and mixing unit, these include: maize, grass silages, and other types of feed stuck. Other types of dry or wet plants may also be processed, such as organic or eco-friendly biomass, such as rice straw, elephant grass, e.g. pennisetum purpureum, saccharum ravennae, miscanthus fuscus, and biomass from willow trees. The exemplified chopper and mixer is from the Italian company SEKO, and is a Chopping-Mixing-Wagon from the Samurai 5 series. Figure 6 shows in more details the chopping and mixing devices that are used by SEKO.

The moveable system according to the present invention, advantageously has dimensions making it suitable for processing high volumes of harvested biomass, and at the same time has dimensions making the system easy to be moved around and used for transport over potentially long distances. Advantageously, a chopper and mixer has a volume capacity between 1 to 50 m³, more preferably between 3 to 40 m³, or 10 to 20 m³, and most preferably is from 5 to 30 or 5 to 33 m³. Thus, advantageously, a chopper and mixer has a volume capacity from 5 to 30, or 5 to 33 m³.

In an embodiment of the invention, the moveable system comprises a chopper and mixer unit with a volumen capacity between 5 to 30, or 5 to 33 m³. In a further embodiment of the invention, the system is adapted for mobile and/or self-propelled or trailed operation. In another embodiment, the system is adapted for stationary operation.

In the embodiment shown in Figure 12, the cut and mixed biomass is transferred to a buffer and feeder unit **8,** by a first transport unit **7.** Advantageously, the first transport unit is of a conveyor type, such as a conveyor selected from the group of: screw-, chain-, band or pneumatic conveyors, and more preferably is a band conveyor. Further advantageously, the first transport unit **7** has an inclination between 0-90 degrees, more preferably between 10-45 degrees, and most preferably between 25-35 degrees.

The buffer and feeder unit **8** is feeding the cut and mixed biomass in desired amounts into the grinder unit **9.**

Figure 3 shows an embodiment of a grinder unit **9.** In the grinder unit **9** the biomass is broken down into smaller and uniform size. This facilitates that the biomass may be further compacted to have an exposed surface area, which is optimal for the further chemical processing step to convert the biomass.

From the grinder **9** the grinded biomass is fed into a buffer storage and/or transport unit **16** by the third transport unit **10,** as illustrated in Figures 10 and 12.

Advantageously, the third transport unit is of a conveyor type, such as a conveyor selected from the group of: screw-, chain-, band or pneumatic conveyors, and more preferably is a band conveyor. Further advantageously, the third transport unit **10** has an inclination between 0-90 degrees, more preferably between 10-45 degrees, and most preferably between 25-35 degrees.

The buffer storage and transport unit **16** may be any type of place suitable for storage and/or transport. In an embodiment, the buffer storage and transport unit **16** is selected from the group of: tip wagon, container, and truck trailer, such as a truck trailer equipped with walking floor.

For the moveable system to be operated at any place, the system **100** further advantageously comprises a control unit, such as a panel (SRO) **11,** and a power unit **12.** Advantageously, the power unit is adapted to the volumes of biomass that need to be processed. For example, the power unit may have a capacity of circa 200 kW, which may be obtained from a tractor with 150 HP on diesel or preferably on biogas, biomethane, or any mixtures therof, such as a mix of biogas and diesel, or a mix of biomethane and diesel.

In an embodiment of the invention, the total power needed corresponds to 42 kWh per 1000 kg dry matter in the biomass to be treated. The capacity on a movable system **100** according to the present invention may be in the range of 3 to 4 ton of dry matter per hour, or 3 to 6 ton of dry matter per hour.

In an embodiment of the invention, the power supply unit has a capacity between 100 to 500 kW, more preferably from 200 kW to 350 kW. In a further embodiment, the power supply unit is configured to generate between 20 to 60 kWh per 1000 kg harvested biomass, more preferably between 30 to 50 kWh, such as 42 kWh.

The movable system **100** may be operated by one person, where the person by use of loader tractor **2** feeds the feeder unit **3** with harvest units. In this case straw with 4500 ton dry matter can be treated within 1500 operation hours. The mobile system may also be operated by two persons, whereby straw with 9000 ton of dry matter may be treated within 3000 hours operation.

### System in combination with a biogas plant

Converting biomass to a substrate for biogas involves the chemical processing step of treating the biomass with a steam comprising nitrogen to separate the fermentable sugars from the lignin within the biomass. The efficiency of the nitrogen steaming process will thus depend on the contact time and degree of contact surface between the steam and the biomass. The contact surface will depend on the size and shape (and thus inherently the surface area of the biomass) as well as the compaction degree (i.e. the access of the steam to the surface of the biomass).

As shown in Figure 10, the grinded biomass may be transferred from the buffer storage and transport unit **16** to a biogas plant **17,** where the grinded biomass is unloaded in unloader/loader units **18** and loaded into a buffer and feeder unit **19** as illustrated in Figure 14.

Figure 14 shows an embodiment of a mobile, or moveable, system **100** in fluid communication with one or more biogas plants **17.** From the buffer store and transport unit **16** the grinded biomass is by use of the unloader / loader unit **18** loaded in buffer and feeder unit **19.** From here it is fed in to the N-steamer **20** that in parallel is supplied with N-steam from the dryer **26** and the N-stripper **29.** From the N-steamer, the steamed material may be fed to a biogas plant **17** in parallel with other biomass that is taken in via bio-mixer **22** and power feed **21.**

As also shown in Figure 14, the nitrogen steaming unit may be fed with biomass from one or more storages, such as the buffer storage and transport unit **16** in fluid communication with the movable system **100** unit, and a second buffer storage and transport unit **16** comprising other sources of grinded biomass that may further be added to the bio-mixer **22** and from there via the power feed **21** added to the biogas plant **17.**

### Stationary systems

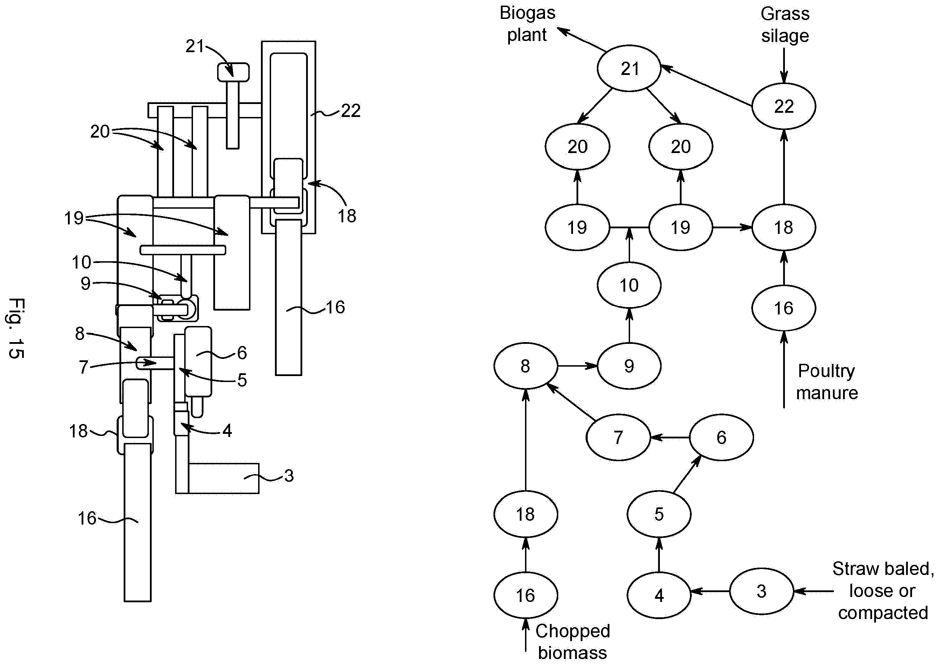

In an alternative embodiment of the invention, the system **100** may be a stationary unit, optionally directly connected to a biogas plant **17.** In this case, the third transport unit **10** load the grinded biomass directly into the buffer and feeder unit **19** as shown in Figure 15. In this case, the power supply **12** can be either directly or a separate motor-generator plant on biogas.

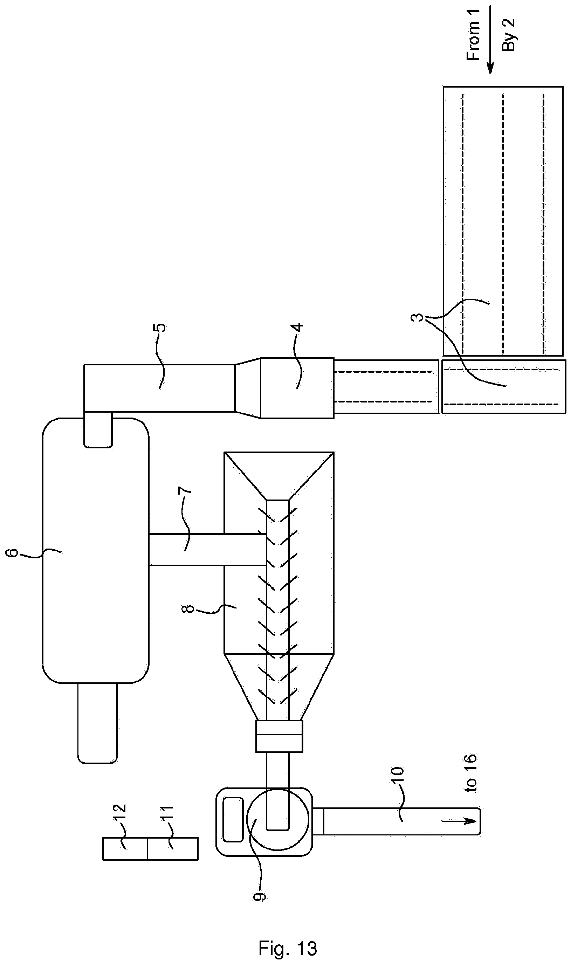

Figure 13 shows an embodiment of a stationary system **100** according to the present invention, and the components comprised within the system and the interaction between them.

The capacity of a stationary unit may be 5 ton dry matter per hour or more, such as between 5 to 10 ton dry matter per hour.

Advantageously, the components may be adapted to the stationary system. For example, advantageously, the cutting and mixing unit **6** for a stationary system and plant is comparable to the unit exemplified in Figure 6.

### Moveable system for grass

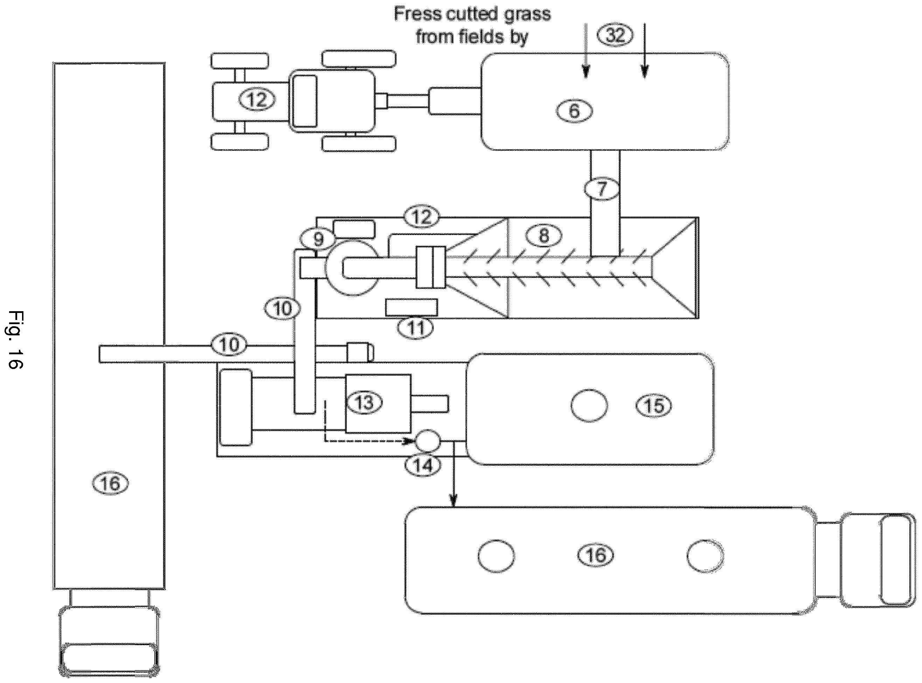

The moveable system according to the present invention may be particularly suitable for processing fresh grass into grass juice for green protein production. Figure 16 shows an embodiment of the invention adapted for processing fresh grass into grass juice for green protein production. The grass is collected and grinded in a similar manner as illustrated in Figure 11, and subsequently transferred to a screw press to produce the extract of protein liquid.

The movable system **100** is used for extracting juice for green protein from grass as illustrated in Figure 16. Advantageously the system is placed in the field, and the extract produced decentral in the field. The extract is subsequently transported to a protein plant **33,** and the residual green mass cake may be transported to a feed stuff plant **34** or biogas plants **17.**

As shown in Figure 16, the grass is collected fresh from field by tractor driven or self-propelled units that is well known equipment for harvesting green mass for silage. The fresh collected grass is added directly to the grinder unit **9** via the cutting and mixing unit 6. The grinded grass is then led to a screw press **13** where the juice is pressed out, collected and by pump **14** led to temporary store for grass juice **15** and from there by road tanker transported to the grass juice processing plant (green protein) **33.** The press cake is led directly by the screw press **13** to a screw or band conveyor / transport unit **10** and loaded in buffer storage and transport units **16** and transferred to feed stuff plant **34** or biogas plants **17.**

Advantageously, the system comprises one or more eliminator unit(s), configured for separating the grass from other materials, such as rope, stone, metal, and other heavy particles. Advantagously, the buffer and feeder unit is connected with an eliminator unit. For example, the buffer and feeder unit advantageously is fluidly connected with an eliminator unit, such that stone, rope and other heavy particles are removed before entering the grinder. The eliminator may for example be mounted at the top of the buffer and feeder unit, and be a top-mounted eliminator.

In a preferred embodiment of the disclosure, the buffer and feeder unit **8** comprises a top-mounted eliminator for separating the biomass from other materials, such as rope and stones, metal and other heavy particles.

Figure 7 shows a more detailed embodiment of a single screw press **13** and figure 8 shows an embodiment of a twin screw press **13.**

Figure 7 shows a single screw press for separation of fresh cuttet grass, grinded grass, or other types of biomass into a liquid fraction, or juice fraction, and a fiber fraction, or press cake fraction. (A) shows the part of the screw press where biomass is fed in from the grinder unit and (B) shows the part of the screw press where the fiber or press cake exit the screw press unit with a dry matter content of up to 35%.

Figure 8 shows a twin screw press for separation of fresh cuttet grass, grinded grass, or other types of biomass into a liquid fraction or juice and a fiber fraction or press cake. (A) shows a twin screw press unit seen from outside, (B) shows the two screws having opposite rotation directions, where one is turning left and the other is turning right, and (C) shows the filter with holes for elimation of the juice. The twin screw press facilitates that the fiber or press cake exit the twin screw press unit with a dry matter content of up to 50 wt%.

In a preferred embodiment, the mobile or moveable system is used for extracting green protein from grass. Advantageously the system is placed in the field, and the extract produced decentrally in the field. The extract is subsequently transported to a protein plant, and the residual green mass may be transported to a green pellet plant or a biogas plant.

Thus, for processing harvested grass to grass juice for green protein production, it is advantageous that the third transport unit **10** is configured to separate the liquid juice from the solid remains. This may be obtained by the third transport unit comprising, or consisting of, a screw press, such as a single screw press or a twin screw press.

In an embodiment of the invention, the third transport unit **10** is configured to separate solid and liquid biomass during the transport of the biomass. In a further embodiment, the third transport unit **10** comprises a screw press **13** configured for separating the grinded product into a liquid and solid product. In a further embodiment, the screw press is selected from the group of: single screw press, twin screw press, and combinations thereof.

### Reference numbers:

- 1: - temporary store for baled, loose or compacted biomass
- 2: - loader
- 3: - feeder for straw in bales, loose or compacted
- 4: - eliminator - ropes, stones, metal and heavy particles
- 5: - second transport unit
- 6: - cutting and mixing unit
- 7: - first transport unit
- 8: - buffer and feeder unit
- 9: - grinder unit
- 10: - third transport unit
- 11: - control panel (SRO)
- 12: - power supply / motor-generator plant / tractor
- 13: - screw press
- 14: - pump
- 15: - temporary store for grass juice
- 16: - buffer storage and transport unit
- 17: - biogas plant
- 18: - unloader/loader unit
- 19: - buffer and feeder unit
- 20: - N-steamer unit
- 21: - power feed
- 22: - bio mixer
- 23: - heater unit
- 24: - separation / screw press
- 25: - fiber / solid outlet
- 26: - dryer unit
- 27: - reject liquid outlet
- 28: - water ring pumps
- 29: - N-stripper
- 30: - N-absorber
- 31: - compactor / pellet press
- 32: - green harvester and collector wagon
- 33: - grass juice processing plant (green protein)
- 34: - feed stuff plant
- 35: - biomass inlet
- 36: - biomass solid outlet
- 37: - biomass liquid outlet
- 38: - screw snail
- 39: - mesh
- 40: - pressure means
- 41: - disc
- 42: - end channel

## Claims

1. A system (100) for processing an agricultural waste biomass product into a grinded product, comprising:
- a cutting and mixing unit (6) configured for cutting and mixing harvested biomass to a first uniform size and blend,
- a grinder unit (9) configured for cutting biomass to a second uniform size, wherein the cutting and mixing unit is in fluid communication with the grinder unit through a first transport unit (7) configured for transporting the biomass from the cutting and mixing unit to the grinder unit, and
**characterized in that** the grinder unit comprises two or more rotating wheels, said rotating wheels having rotation axes with an angular displacement.

2. The system according to claim 1, wherein the second uniform size is smaller than the first uniform size.

3. The system according to any of the preceding claims, wherein the cutting and mixing unit (6) comprises two rotating screws, wherein the screws are configured to rotate in opposite directions, and preferably the cutting and mixing unit is selected from the group of chopper and chopper and mixer, and/or wherein the cutting and mixing unit (6) has a volume capacity between 1 to 50 m³, more preferably between 3 to 40 m³, or 10 to 20 m³, and most preferably from 5 to 30 m³, or from 5 to 33 m³, and/or wherein the cutting and mixing unit (6) is in fluid communication with a second transport unit (5) adapted as a feedstock control means, such as a feeder unit or a silage tiller, and optionally is a band conveyor with an inclination between 0-45 degrees, more preferably between 10-30 degrees, and most preferably between 15-20 degrees.

4. The system according to any of the preceding claims, wherein the first transport unit (7) is a conveyor, preferably selected from the group of: screw-, chain-, flight-, and pneumatic conveyors, and more preferably is a screw conveyor.

5. The system according to any of the preceding claims, wherein the grinder unit (9) is not a hammermill, and/or wherein the grinder unit (9) is configured to operate on moist or wet biomass, and/or wherein the grinder unit (9) comprises two or more rotating wheels configured for cutting biomass, preferably comprising three rotating wheels, and a matrix comprising multiple openings configured for transferring grinded biomass with a size below the size of the openings, preferably wherein the openings have a diameter between 1 to 40 mm, more preferably between 6 to 24 mm, and most preferably between 12 to 16 mm.

6. The system according to any of the preceding claims, further comprising a third transport unit (10), configured for transferring the grinded biomass from the outlet of the grinder unit, preferably the third transport unit is selected from the group of: screw-, chain-, band or pneumatic conveyors, and more preferably is a band conveyor with an inclination between 0-90 degrees, more preferably between 10-45 degrees, and most preferably between 25-35 degrees.

7. The system according to any of the preceding claims, wherein the first transport unit (7) is a conveyor with an inclination between 0-90 degrees, more preferably between 10-45 degrees, and most preferably between 25-35 degrees.

8. The system according to any of the preceding claims, wherein the system is configured to be mobile, moveable, or stationary, and optionally the system is configured to be self-propelled and/or trailed.

9. The system according to any of the preceding claims, further comprising a buffer and feeder unit (8) configured for supplying the biomass to the grinder unit, and/or further comprising a control unit (11), such as a a panel or a SRO, and/or further comprising a power supply unit (12), such as a motor-generated plant or a tractor, and/or further comprising a feeder unit (3) configured for transferring straw in bales or loose, and/or further comprising an eliminator unit (4) configured for separating the biomass from other materials, such as rope and stones.

10. The system according to any of the preceding claims, configured to be moveable and comprising a first transport unit (5), a buffer and feeder unit (8), a third transport unit (10), a control unit (11), and a power supply unit (12), and optionally further comprising a feeder unit (3), and an eliminator unit (4), wherein preferably the eliminator unit comprises one or more sections, and wherein at least one section comprises magnetic means or means for providing an air flow means.

11. The system according to any of claims 9-10, wherein the power supply unit has a capacity between 100 to 500 kW, more preferably from 200 kW to 350 kW, and optionally wherein the power supply unit is configured to generate between 20 to 60 kWh per 1000 kg harvested biomass, more preferably between 30 to 50 kWh, such as 42 kWh.

12. The system according to any of the preceding claims, wherein the first transport unit (7) is configured to separate solid and liquid biomass during the transport of the biomass.

13. The system according to any of claims 6-12, wherein the third transport unit (10) is configured to separate solid and liquid biomass during the transport of the biomass, optionally wherein the third transport unit (10) comprises a screw press (13) configured for separating the grinded product into a liquid and solid product.

14. A method for processing an agricultural waste biomass product into a grinded product, comprising the steps of:
- providing a harvested agricultural waste biomass,
- cutting and mixing the biomass into a first uniform size and blend within a cutting and mixing unit (6) configured for cutting and mixing harvested biomass to a first uniform size and blend,
- grinding the cut and mixed biomass into a second uniform size within a grinder unit (9) configured for cutting biomass to a second uniform size, wherein the grinder unit comprises two or more rotating wheels, said rotating wheels having rotation axes with an angular displacement, whereby a grinded product suitable as a substrate for a biogas plant and/or as primary growth substrate for a plant, such as mushroom, and/or as substrate for producing green protein extract is produced.

15. Use of the system according to any of claims 1-13 for processing an agricultural waste biomass product into a grinded product, suitable for a substrate for a biogas plant and/or as primary growth substrate for a plant, such as mushroom, and/or as substrate for producing green protein extract, or as feedstock unit for one or more biogas plant(s).

## Patentansprüche

1. System (100) zum Verarbeiten eines landwirtschaftlichen Biomasseabfallprodukts zu einem Mahlprodukt, umfassend:
- eine Schneid- und Mischeinheit (6), die dazu konfiguriert ist, geerntete Biomasse in eine erste einheitliche Größe und Mischung zu schneiden und zu mischen,
- eine Mahleinheit (9), die dazu konfiguriert ist, Biomasse in eine zweite einheitliche Form zu schneiden, wobei die Schneid- und Mischeinheit durch eine erste Transporteinheit (7), die dazu konfiguriert ist, die Biomasse von der Schneid- und Mischeinheit zu der Mahleinheit zu transportieren, in Fluidkommunikation mit der Mahleinheit steht, und
**dadurch gekennzeichnet, dass** die Mahleinheit zwei oder mehr drehende Räder umfasst, wobei die drehenden Räder Drehachsen mit einer Winkelverschiebung aufweisen.

2. System nach Anspruch 1, wobei die zweite einheitliche Größe kleiner als die erste einheitliche Größe ist.

3. System nach einem der vorhergehenden Ansprüche, wobei die Schneid- und Mischeinheit (6) zwei drehende Schnecken umfasst, wobei die Schnecken dazu konfiguriert sind, sich in entgegengesetzte Richtungen zu drehen, und vorzugsweise die Schneid- und Mischeinheit aus der Gruppe aus Häcksler und Häcksler und Mischer ausgewählt ist und/oder wobei die Schneid- und Mischeinheit (6) ein Fassungsvermögen zwischen 1 und 50 m³, noch besser zwischen 3 und 40 m³ oder 10 und 20 m³ und am besten von 5 bis 30 m³ oder von 5 bis 33 m³ aufweist und/oder wobei die Schneid- und Mischeinheit (6) in Fluidkommunikation mit einer zweiten Transporteinheit (5) steht, die als Zuführungssteuereinheit ausgelegt ist, wie etwa als Zuführeinheit oder Silagefräse, und optional ein Förderband mit einer Neigung zwischen 0-45 Grad, noch besser zwischen 10-30 Grad und am besten zwischen 15-20 Grad ist.

4. System nach einem der vorhergehenden Ansprüche, wobei die erste Transporteinheit (7) eine Fördervorrichtung ist, vorzugsweise aus der folgenden Gruppe ausgewählt ist: Förderschnecke, Förderkette, Mitnehmer-Fördervorrichtung und pneumatische Fördervorrichtung, und am besten eine Förderschnecke ist.

5. System nach einem der vorhergehenden Ansprüche, wobei die Mahleinheit (9) keine Hammermühle ist und/oder wobei die Mahleinheit (9) für die Handhabung von feuchter oder nasser Biomasse konfiguriert ist und/oder wobei die Mahleinheit (9) zwei oder mehr drehende Räder, die zum Schneiden von Biomasse konfiguriert sind und vorzugsweise drei drehende Räder umfassen, und eine Matrize umfasst, die mehrere Öffnungen umfasst, die dazu konfiguriert sind, gemahlene Biomasse mit einer Größe unterhalb der Größe der Öffnungen weiterzugeben, wobei die Öffnungen einen Durchmesser zwischen 1 und 40 mm, noch besser zwischen 6 bis 24 mm und am besten zwischen 12 und 16 mm aufweisen.

6. System nach einem der vorhergehenden Ansprüche, ferner umfassend eine dritte Transporteinheit (10), die dazu konfiguriert ist, die gemahlene Biomasse von dem Auslass der Mahleinheit weiterzugeben, wobei vorzugsweise die dritte Transporteinheit aus der folgenden Gruppe ausgewählt ist: Förderschnecke, Förderkette, Förderband oder pneumatische Fördervorrichtung, und insbesondere ein Förderband mit einer Neigung zwischen 0-90 Grad, noch besser zwischen 10-45 Grad und am besten zwischen 25-35 Grad ist.

7. System nach einem der vorhergehenden Ansprüche, wobei die erste Transporteinheit (7) eine Fördervorrichtung mit einer Neigung zwischen 0-90 Grad, noch besser zwischen 10-45 Grad und am besten zwischen 25-35 Grad ist.

8. System nach einem der vorhergehenden Ansprüche, wobei das System mobil, beweglich oder stationär konfiguriert ist und optional das System als selbstangetrieben und/oder gezogen konfiguriert ist.

9. System nach einem der vorhergehenden Ansprüche, ferner umfassend eine Zwischenlager- und Zuführungseinheit (8), die dazu konfiguriert ist, die Biomasse der Mahleinheit zuzuführen, und/oder ferner umfassend eine Steuereinheit (11), wie etwa eine Schalttafel oder SRO, und/oder ferner umfassend eine Leistungsversorgungseinheit (12), wie etwa eine elektromotorbetriebene Anlage oder eine Zugmaschine, und/oder ferner umfassend eine Zuführungseinheit (3), die dazu konfiguriert ist, Stroh in Ballen oder lose weiterzugeben, und/oder ferner umfassend eine Beseitigungseinheit (4), die dazu konfiguriert ist, die Biomasse von anderen Materialien, wie etwa Seil oder Steinen, zu trennen.

10. System nach einem der vorhergehenden Ansprüche, das beweglich konfiguriert ist und eine erste Transporteinheit (5), eine Zwischenlager- und Zuführungseinheit (8), eine dritte Transporteinheit (10), eine Steuereinheit (11) und eine Leistungsversorgungseinheit (12) umfasst und optional ferner eine Zuführungseinheit (3) und eine Beseitigungseinheit (4) umfasst, wobei vorzugsweise die Beseitigungseinheit einen oder mehrere Teilabschnitte umfasst und wobei mindestens ein Teilabschnitt Magnetmittel oder Mittel zum Bereitstellen eines Luftstroms umfasst.

11. System nach einem der Ansprüche 9-10, wobei die Leistungsversorgungseinheit eine Kapazität zwischen 100 und 500 kW, insbesondere von 200 kW bis 350 kW aufweist und wobei optional die Leistungsversorgungseinheit dazu konfiguriert ist, zwischen 20 und 60 kWh pro 1000 kg geernteter Biomasse zu generieren, insbesondere zwischen 30 und 50 kWh, wie etwa 42 kWh.

12. System nach einem der vorhergehenden Ansprüche, wobei die erste Transporteinheit (7) dazu konfiguriert ist, während des Transports der Biomasse feste und flüssige Biomasse zu trennen.

13. System nach einem der Ansprüche 6-12, wobei die dritte Transporteinheit (10) dazu konfiguriert ist, während des Transports der Biomasse feste und flüssige Biomasse zu trennen, wobei optional die dritte Transporteinheit (10) eine Schneckenpresse (13) umfasst, die dazu konfiguriert ist, das Mahlprodukt in ein flüssiges und ein festes Produkt aufzutrennen.

14. Verfahren zum Verarbeiten eines landwirtschaftlichen Biomasseabfallprodukts zu einem Mahlprodukt, das die folgenden Schritte umfasst:
- Bereitstellen eines geernteten landwirtschaftlichen Biomasseabfalls,
- Schneiden und Mischen der Biomasse in eine einheitliche Größe und Mischung in einer Schneid- und Mischeinheit (6), die dazu konfiguriert ist, die geerntete Biomasse in eine einheitliche Größe und Mischung zu schneiden und zu mischen,
- Mahlen der geschnittenen und vermischten Biomasse in eine zweite einheitliche Größe in einer Mahleinheit (9), die dazu konfiguriert ist, Biomasse in eine zweite einheitliche Größe zu schneiden, wobei die Mahleinheit zwei oder mehr drehende Räder umfasst, wobei die drehenden Räder Drehachsen mit einer Winkelverschiebung aufweisen, wodurch ein Mahlprodukt, das als Substrat für eine Biogasanlage und/oder als primäres Wachstumssubstrat für eine Pflanze, wie etwa Pilze, und/oder als Substrat zum Erzeugen eines grünen Proteinextrakts geeignet ist, ist verarbeitet.

15. Verwendung des Systems nach einem der Ansprüche 1-13 zur Verarbeitung eines landwirtschaftlichen Biomasseabfallprodukts zu einem Mahlprodukt, das für ein Substrat für eine Biogasanlage und/oder als primäres Wachstumssubstrat für eine Pflanze, wie etwa Pilze, und/oder als Substrat zum Erzeugen eines grünen Proteinextrakts geeignet ist, oder als Zuführeinheit für eine oder mehrere Biogasanlagen.

## Revendications

1. Système (100) pour transformer un produit de biomasse de déchets agricoles en un produit broyé, comprenant :
- une unité de coupe et de mélange (6) conçue pour couper et mélanger une biomasse récoltée à une première taille et un premier mélange uniformes,
- une unité de broyage (9) conçue pour couper la biomasse selon une seconde taille uniforme, dans lequel l'unité de coupe et de mélange est en communication fluidique avec l'unité de broyage par le biais d'une première unité de transport (7) conçue pour transporter la biomasse de l'unité de coupe et de mélange jusqu'à l'unité de broyage, et
**caractérisé en ce que** l'unité de broyage comprend deux roues rotatives ou plus, lesdites roues rotatives présentant des axes de rotation ayant un déplacement angulaire.

2. Système selon la revendication 1, dans lequel la seconde taille uniforme est plus petite que la première taille uniforme.

3. Système selon l'une quelconque des revendications précédentes, dans lequel l'unité de coupe et de mélange (6) comprend deux vis rotatives, dans lequel les vis sont conçues pour tourner dans des directions opposées, et de préférence l'unité de coupe et de mélange est sélectionnée à partir du groupe constitué d'un hacheur et d'un hacheur et mélangeur, et/ou dans lequel l'unité de coupe et de mélange (6) présente une capacité volumique entre 1 et 50 m³, de préférence entre 3 et 40 m³, ou 10 et 20 m³, et de manière préférée entre toutes de 5 à 30 m³, ou de 5 à 33 m³, et/ou dans lequel l'unité de coupe et de mélange (6) est en communication fluidique avec une deuxième unité de transport (5) adaptée en tant que moyen de commande de charge d'alimentation, tel qu'une unité de chargement ou une désileuse, et optionnellement est un convoyeur à bande présentant une inclinaison entre 0 et 45 degrés, de préférence entre 10 et 30 degrés, et de manière préférée entre toutes entre 15 et 20 degrés.

4. Système selon l'une quelconque des revendications précédentes, dans lequel la première unité de transport (7) est un convoyeur, de préférence sélectionné à partir du groupe de : convoyeurs à vis, à chaînes, à raclettes, et pneumatiques, et de préférence est un convoyeur à vis.

5. Système selon l'une quelconque des revendications précédentes, dans lequel l'unité de broyage (9) n'est pas un broyeur à marteaux, et/ou dans lequel l'unité de broyage (9) est conçue pour fonctionner sur une biomasse humide ou mouillée, et/ou dans lequel l'unité de broyage (9) comprend deux roues rotatives ou plus conçues pour couper la biomasse, de préférence comprenant trois roues rotatives, et une matrice comprenant de multiples ouvertures conçues pour transférer une biomasse broyée présentant une taille inférieure à la taille des ouvertures, de préférence dans lequel les ouvertures présentent un diamètre entre 1 et 40 mm, de préférence entre 6 et 24 mm, et de manière préférée entre toutes entre 12 et 16 mm.

6. Système selon l'une quelconque des revendications précédentes, comprenant en outre une troisième unité de transport (10), conçue pour transférer la biomasse broyée depuis la sortie de l'unité de broyage, de préférence la troisième unité de transport est sélectionnée à partir du groupe de : convoyeurs à vis, à chaînes, à bandes ou pneumatiques, et de manière davantage préférée est un convoyeur à bandes présentant une inclinaison entre 0 et 90 degrés, de préférence entre 10 et 45 degrés, et de manière préférée entre toutes entre 25 et 35 degrés.

7. Système selon l'une quelconque des revendications précédentes, dans lequel la première unité de transport (7) est un convoyeur présentant une inclinaison entre 0 et 90 degrés, de préférence entre 10 et 45 degrés, et de manière préférée entre toutes entre 25 et 35 degrés.

8. Système selon l'une quelconque des revendications précédentes, dans lequel le système est conçu pour être mobile, pouvoir être déplacé, ou stationnaire, et optionnellement le système est conçu pour être autopropulsé et/ou remorqué.

9. Système selon l'une quelconque des revendications précédentes, comprenant en outre une unité de mise en tampon et de chargement (8) conçue pour apporter la biomasse à l'unité de broyage, et/ou comprenant en outre une unité de commande (11), telle qu'un panneau ou un SRO, et/ou comprenant en outre une unité d'alimentation électrique (12), telle qu'une installation à moteur-générateur ou un tracteur, et comprenant en outre une unité de chargement, (3) conçue pour transférer la paille en balles ou en vrac, et/ou comprenant en outre une unité d'élimination (4) conçue pour séparer la biomasse d'autres matériaux, tels que de la corde et des pierres.

10. Système selon l'une quelconque des revendications précédentes, conçu pour pouvoir être déplacé et comprenant une première unité de transport (5), une unité de mise en tampon et de chargement (8), une troisième unité de transport (10), une unité de commande (11) et une unité d'alimentation électrique (12), et optionnellement comprenant en outre une unité de chargement (3), et une unité d'élimination (4), dans lequel de préférence l'unité d'élimination comprend une ou plusieurs sections, et dans lequel au moins une section comprend un moyen magnétique ou un moyen pour fournir un moyen d'écoulement d'air.

11. Système selon l'une quelconque des revendications 9-10, dans lequel l'unité d'alimentation électrique présente une capacité entre 100 et 500 kW, de préférence de 200 kW à 350 kW, et optionnellement dans lequel l'unité d'alimentation électrique est conçue pour générer entre 20 et 60 kWh par 1000 kg de biomasse récoltée, de préférence entre 30 et 50 kWh, tel que 42 kWh.

12. Système selon l'une quelconque des revendications précédentes, dans lequel la première unité de transport (7) est conçue pour séparer la biomasse liquide et la biomasse solide durant le transport de la biomasse.

13. Système selon l'une quelconque des revendications 6 à 12, dans lequel la troisième unité de transport (10) est conçue pour séparer la biomasse liquide et la biomasse solide durant le transport de la biomasse, optionnellement dans lequel la troisième unité de transport (10) comprend une presse à vis (13) conçue pour séparer le produit broyé en un produit solide et un produit liquide.

14. Procédé pour transformer un produit de biomasse de déchets agricoles en un produit broyé, comprenant les étapes de :
- fourniture d'une biomasse de déchets agricoles récoltée,
- coupe et mélange de la biomasse selon une première taille et un premier mélange uniformes dans une unité de coupe et de mélange (6) conçue pour couper et mélanger la biomasse récoltée à une première taille et un premier mélange uniformes,
- broyer la biomasse coupée et mélangée selon une seconde taille uniforme dans une unité de broyage (9) conçue pour couper la biomasse à une seconde taille uniforme, dans lequel l'unité de broyage comprend deux roues rotatives ou plus, lesdites roues rotatives présentant des axes de rotation ayant un déplacement angulaire, moyennant quoi un produit broyé approprié en tant que substrat pour une installation biogaz et/ou en tant que substrat de croissance primaire pour un végétal, tel qu'un champignon, et/ou en tant que substrat pour produire un extrait protéique vert est obtenu.

15. Utilisation du système selon l'une quelconque des revendications 1 à 13 pour transformer un produit de biomasse de déchets agricoles en un produit broyé, approprié en tant que substrat pour une installation biogaz et/ou en tant que substrat de croissance primaire pour un végétal, tel qu'un champignon, et/ou en tant que substrat pour produire un extrait protéique vert, ou comme unité de charge d'alimentation pour une ou plusieurs installation(s) biogaz.
